# EUROPEAN PATENT SPECIFICATION

(11) **EP 1 585 835 B1**
(45) Date of publication and mention of the grant of the patent: **18.04.2007**
(21) Application number: 03811797.4
(22) Date of filing: 19.11.2003
(51) Int. Cl.: C12Q 1/68

(54) **Nucleic acid probes and method for detecting a target nucleic acid sequence comprising two or more cis-located nucleic acid regions**
Nukleinsäuresonden und Verfahren zum Nachweis einer Zielnukleinsäuresequenz mit zwei oder mehr cis-Nukleinsäurebereichen
Sondes d'acides nucléiques et procédé pour détecter une séquence cible d'acides nucléiques comprenant deux regions d'acides nucléiques ou plus, situées en cis

(30) Priority: 22.11.2002 GB 0227274
(43) Date of publication of application: 19.10.2005
(73) Proprietor: Dynal Biotech Limited, Bromorough, Wirral CH62 3QL (GB)
(72) Inventor: CROSBY, Ian, Wirral CH63 0NE (GB); MICHAEL, Bunce, Caerwys, Flintshire CH7 5BQ (GB)
(74) Representative: Davies, Punita Shah
(86) International application number: PCT/GB2003/005012
(87) International publication number: WO 2004/048611

(56) References cited:
- WO-A-03/027309
- US-A- 4 835 098
- US-A1- 2001 019 825
- MARSH S G ET AL: "Nomenclature for factors of the HLA system, 2000." HUMAN IMMUNOLOGY. UNITED STATES APR 2001, vol. 62, no. 4, April 2001 (2001-04), pages 419-468, XP002273667 ISSN: 0198-8859
- BEAUCAGE S L ET AL: "THE SYNTHESIS OF MODIFIED OLIGONUCLEOTIDES BY THE PHOSPHORAMIDITE APPROACH AND THEIR APPLICATIONS" TETRAHEDRON, ELSEVIER SCIENCE PUBLISHERS, AMSTERDAM, NL, vol. 49, no. 28, 1993, pages 6123-6194, XP001008840 ISSN: 0040-4020
- BEAUCAGE S L ET AL: "THE FUNCTIONALIZATION OF OLIGONUCLEOTIDES VIA PHOSPHORAMIDITE DERIVATIVES" TETRAHEDRON, ELSEVIER SCIENCE PUBLISHERS, AMSTERDAM, NL, vol. 49, no. 10, 1993, pages 1925-1963, XP000857919 ISSN: 0040-4020

## Description

The present invention relates to probes, methods of detection and diagnosis, and apparatus for use in such methods. In particular, the present invention relates to nucleic acid, especially oligonucleotide, probes and to methods and apparatus for the detection of the presence or absence of non-contiguous cis-located nucleic acid sequences and polymorphisms therein.

Two or more nucleic acid polymorphisms on a single molecule or strand are said to be *cis*-located and these occur naturally in nucleic acid sequences, particularly in alleles belonging to the human leucocyte antigen (HLA) system. As detailed by Bodmer et al (Tissue Antigens (1999) 53 407-446), the HLA locus is the most polymorphic in the human genome. There are currently over 240 HLA-A alleles, 478 HLA-B, 116 HLA-C, 99 HLA-DPB1, 52 DQB1 and 305 DRB 1 alleles, with new alleles being discovered continuously. All HLA-A, -B, and -C alleles have similar sequences made up of blocks of polymorphisms, or sequence motifs. The differing alleles are generally made up of recombinations of these sequence motifs. The same holds for class II loci such as DRB1, DRB3, DRB4, DRB5 and DPB1.

Many methods exist for HLA genotyping. One common method utilises target DNA amplification by polymerase chain-reaction (PCR) followed by detection of polymorphic DNA sequences using sequence-specific oligonucleotide probes (SSO) (Saiki RK et al, Nature. 1986 Nov 13-19;324(6093):163-6). The SSO probes hybridised to target DNA may be detected by, for example, colorimetric, radioactive or fluorescent methods. In HLA genotyping the initial amplification is normally generic, but may comprise a mosaic of amplifications which when used together amplify all possible alleles of a given locus. SSO techniques were first applied to HLA genotyping (HLA-DQA1) by Saiki et al (Saiki RK et al, Nature. 1986 Nov 13-19;324(6093):163-6). Subsequently SSO reverse blot techniques (Erlich H et al, Eur J Immunogenet. 1991 Feb-Apr;18(1-2):33-55) were developed. Reverse methods depend on the incorporation of a chemical label such as biotin in the initial generic PCR amplification, usually via labeled amplification primers. In the reverse dot blot, the SSO probes are bound to a solid support membrane leaving the detection end of the probe free to interact with target DNA. On a single membrane many different probes can be bound which would theoretically contain all of the polymorphisms required to genotype an individual at any given locus. When labeled DNA target is applied to the reverse dot blot membrane the DNA will only hybridise to the probes that are matched in DNA sequence. Hybridised biotin-labeled products are detected by the addition of a reporter molecule that induces a colour change in the substrate. Whether a probe will hybridise specifically to a target DNA sequence is dependent upon the amount of probe-target mismatching, mismatch position relative to the probe and probe length and is largely dependent upon the conditions for the hybridisation (such as temperature and salt concentration).

HLA alleles are constructed from a patchwork of polymorphic DNA motifs that are shared by other alleles. It is not possible to discriminate between certain sets of alleles using conventional SSO techniques due to these shared motifs and the fact that most animals are diploid, in that we all have two copies of each locus, i.e. two HLA-A alleles, two HLA-B etc. As a consequence of shared motifs, a single probe is likely to be capable of detecting a number of different alleles that compromise the specificity of the probe by detecting a large number of alleles rather than a small number of alleles or being specific for one allele.

In addition, using conventional SSO techniques utilising a mixture of probes (one for each spatially separated target polymorphism), it is not possible to determine whether the motifs are present or not on the same allele since each of the probes will hybridise to different alleles if the two alleles present in the DNA both contain one or both of the target polymorphisms.

A theoretical possibility to overcome such problems is to design a probe which will hybridise with two or more target polymorphisms but not with their intervening nucleic acid sequence. However, in circumstances where the target DNA motifs are spaced apart, designing a single probe with standard matching nucleotides complementary to the spatially separated target DNA motifs and the intervening sequence between them will impact on the probe's hybridisation temperature increasing it beyond normal design constraints for conventional SSO techniques where a probe to target DNA hybridization temperature are normally within the range 35°C-65°C.

U.S. Pat. App. No. 20010019825 discloses a method of amplifying DNA for detecting target nucleic acid sequences with diagnostic primers including primer regions and probe regions which are complementary to target and reference regions respectively on a sample nucleic acid strand. Optionally, there is provided a region on the diagnostic primer that is separated by a spacer region of nucleic acid.

U.S. Pat. App. No. 20020042077 discloses partially non-hybridising oligonucleotides that contain two or more hybridising segments, with any two hybridising segments separated by a non-hybridising spacer segment. The art in this application is the design of probes with multiple hybridising regions, but not specifically to detect *cis*-located polymorphisms for the purpose of increasing a given probe's specificity for an allele or group of alleles sharing the two or more polymorphisms.

Another major problem associated with probes that comprise two hybridising segments and a non-hybridising spacer segment is that the non-hybridising spacer, being composed either of nucleic acid or a compound with similar properties to that of nucleic acid, produces a probe that requires less stringent hybridisation conditions. The reduced stringency is to allow for hybridisation in the presence of mis-matched nucleotides/nucleosides or similar compounds within the spacer segment, which leads to problems with false positives and results that can be hard to interpret.

It is an object of the present invention to alleviate or overcome one or more of the problems associated with the prior art.

In accordance with a first aspect of the present invention there is provided a nucleic acid probe as defined by appending claim 1.

The invention relates to probes capable of detecting non-contiguous cis-located nucleic acid sequences which are characteristic of certain alleles including those relating to the human leukocyte antigen (HLA), and other genes within the major histocompatibility complex (MHC) which is of interest in the field of human transplantation and disease. However, the probe of the invention is not restricted to be determinative of genes within the MHC but can be applied to any allelic system in which the alleles have two or more *cis*-located regions to be detected. For example the probe according to the invention may be designed to target other polymorphic genes including, but not restricted to, thiopurine methyl transferases (TPMT), heamochromatosis gene (HFE), tumour necrosis factor (TNF); lymphotoxin (LT), mannose binding lectin (MBL), ABO and other blood grouping systems such as Secretor, Duffy and Rhesus, Factor V Leiden, platelet membrane glycoproteins (GPIIIaIIIb/Ib/IX), human platelet antigens (HPA), CC-chemokine receptor 5 (CCRS), interleukin genes and interleukin receptors, chemokine genes and chemokine receptors, cystic fibrosis genes, KIR genes, cluster differentiation antigens (CD), such as CD1, NOTCH genes, TOLL genes, heat shock proteins, xanthidin oxidase (XO), manganese superoxide dismutase (SOD), paraxonase, N-acetyl transferase (NAT-1 and NAT2), cytochrome P450 CYP2D6 (debrisoquine hydroxylase), multidrug resistance gene 1 (MDR1) and cell adhesion molecules such as MICA, MICB, VCAM, ICAM and PECAM.

The target nucleic acid sequence may comprise one or more alleles of a gene. For example the target sequence may comprise one or more alleles of HLA. Examples of other alleles that could be determined by the probe according to the invention include, but are not limited to, TPMT*3a, TNF alleles, referred to as the allelic types -238G/-238A-308G/-308A-376G/-376A (see Knight et al Nature Genet. 22: 145-150,1999), HFE C282Y (Feder et al Proc. Nat. Acad. Sci. - 95:1472-1477,-1998):

The spacer region preferably will not hybridise to any substantial degree with nucleic acid sequences in the target molecule or with other nucleic acid sequences in the sample being probed. This effectively means that the hybridisation conditions under which the probe will hybridise with the target nucleic acid sequence is determined substantially by the nucleotide (or nucleoside) content of the non-contiguous regions of the probe.

The probe of the invention yields a significant advantage over prior art bridged probes in that the length of the spacer region is selected to allow pairwise matching of the non-contiguous regions with *cis*-located non-contiguous motifs in a target allele(s), thus making the probe specific for two or more regions when present on the same allele.

The spacer region may comprise, for example, abasic phosphoroamidite ribose molecules, nucleic acid molecules at least substantially mismatched with the target sequence in the intervening region between the cis-located regions, or may comprise any other nucleotide analogue that does not hybridise, or only hybridizes weakly, with the intervening target DNA sequence and which maintains pairwise complimentarity between the two or more hybridizing regions. Preferably, the spacer region comprises a sequence of molecules, each of which are the same, or a similar, size to a nucleic acid base. Thus, the spacer preferably comprises one or more molecules that are spatially correct, having the same or similar spatial dimensions as a nucleic acid base, such as a purine or a pyrimidine. These bases are known to those in the art and include, but are not limited to adensosine derivatives, guanosine derivatives, cytosine derivatives, thymidine derivatives, locked nucleic acids (LNA), peptide nucleic acids (PNA), amino nucleic acids (ANA), 2-amino-2'-deoxyadenosine (2-amino-2'-dA), 2',3'-aideoxyadenosine (2',3'-ddA),3'-de6Xjadenosine, cordycepin (3'-dA), 7-deaza-2'-deoxyadenosine (7-deaza-2'-dA), 8-bromo-2'-deoxyadenosine (8-Br-2'-dA), N6-methyl-2'-deoxyadenosine (N6-methyl-2'-dA, 2',3'-dideoxycytidine (2',3'-ddC), 5-methyl-2'-deoxycytidine (5-Me-2'-dC), 5-bromo-2'-deoxycytidine (5-Br-2'-dC), 5-iodo-2'-deoxycytidine (5-I-2'-dC), 7-deaza-2'-deoxyguanosine (7-deaza-2'-dG), 8-bromo-2'-deoxyguanosine (8-Br-2'-dG), 4-thio-2'-deoxythymidine (4-thio-dT), 5-C3-carboxy-2'-deoxythymidine (5-C3-carboxy-dT), 5-C6-amino-2'-deoxythymidine (5-C6-amino-dT), inverse-2'-deoxythymidine (inverse-dT), 2'-deoxyuridine (2'-dU), 2,6-diaminopurine-2'-deoxyriboside, 2-aminopurine-2'-deoxyriboside, 6-thio-2'-deoxyguanosine, 7-deaza-2'-deoxyadenosine, 7-deaza-2'-deoxyguanosine, 7-deaza-2'-deoxyxanthosine, 8-bromo-2'-deoxyadenosine, 8-bromo-2'-deoxyguanosine, 8-oxo-2'-deoxyadenosine, 8-oxo-2'-deoxyguanosine, etheno-2'-deoxyadenosine, N6-methyl-2'-deoxyadenosine, 06-methyl-2'-deoxyguanosine, O6-phenyl-2'-deoxyinosine, 2'-deoxypseudouridine, 2'-deoxyuridine, 2-thiothymidine, 4-thio-2'-deoxyuridine, 4-thiothymidine, 4-triazolyl-2'-deoxyuridine, 4-triazolylthymidine, 5'-aminothymidine, 5'-iodothymidine, 5'-O-methylthymidine, 5,6-dihydro-2'-deoxyuridine, 5,6-dihydrothymidine, 5-bromo-2'-deoxycytidine, 5-bromo-2'-deoxyuridine, 5-fluoro-2'-deoxyuridine, 5-hydroxy-2'-deoxyuridine, 5-hydroxymethyl-2'-deoxyuridine, 5-iodo-2'-deoxycytidine, 5-iodo-2'-deoxyuridine, 5-methyl-2'-deoxycytidine, 5-propynyl-2'-deoxycytidine, 5-propynyl-2'-deoxyuridine, carboxy thymidine, N4-ethyl-2'-deoxycytidine, O4-methylthymidine, TMP-F-2'-deoxyuridine, 1-methyladenine, 2-methyladenine, N.sup.6-methyladenine, N.sup.6-isopentyladenine, 2-methylthio-N.sup.6-isopentyladenine, N,N-dimethyladenine, 8-bromoadenine, 2-thiocytosine, 3-methylcytosine, 5-methylcytosine, 5-ethylcytosine, 4-acetylcytosine, 1-methylguanine, 2-methylguanine, 7-methylguanine, 2,2-dimethylguanine, 8-bromo-guanine, 8-chloroguanine, 8-amino guanine, 8-methylguanine, 8-thioguanine, 5-fluoro-uracil, 5-bromouracil, 5-chlorouracil, 5-iodouracil, 5-ethyluracil, 5-propyluracil, 5-methoxyuracil, 5-hydroxymethyluracil, 5-(carboxyhydroxymethyl)uracil, 5-(methylaminomethyl)uracil, 5-(carboxymethylaminomethyl)-uracil, 2-thiouracil, 5-methyl-2-thiouracil, 5-(2bromovinyl)uracil, uracil-5-oxyacetic acid, uracil-5-oxyacetic acid methyl ester, pseudouracil, 1-methylpseudouracil, queosine, inosine, 1-methylinosine, hypoxanthine, xanthine, 2-aminopurine, 6-hydroxyaminopurme, 6-thiopurine and 2,6-diaminopurine, 3'-thioether-2'3'-dideoxynucleoside-5'-triphosphates, 3'-thioamido-2',3'-dideoxynucleoside-5'-triphosphates 3'-alkyl-2',3'-dideoxynucleoside-5'-triphosphates , 3'-urea-2',3'-dideox-ynucleoside-5'-triphosphates, and 3'-thiourea- modified 2',3'-dideoxynucleoside-5'-triphosphates and mismatched purines or pyrimidines.

The spacer region may be interrupted with one or more specific nucleic acids selected to pair with complementary bases on the target nucleic acid sequence, allowing the probe to distinguish mutations or discrete sequences in the region of the target nucleic acid sequence corresponding to the spacer region of the probe on hybridisation. Such a probe may afford greater specificity, particularly where the nucleotide on the target sequence which corresponds to the at least one complementary base is a polymorphism.

The hybridizing regions of the probe comprise nucleic acid regions capable of hybridizing with respective *cis*-located nucleic acid regions on the target sequence. These nucleic acid regions of the probe will often suitably comprise nucleotides but may alternatively or also comprise modified bases (DNA analogues) which may in some cases bind to the target sequence more efficiently than conventional nucleotides. Suitable DNA analogues include, for example, amino nucleic acid (ANA), peptide nucleic acid (PNA) and locked nucleic acid (LNA). The hybridizing nucleic acid regions of the probe may also comprise nucleosides. The nucleotides, nucleosides, and/or analogues thereof which make up the hybridizing regions of the probe are preferably selected for the property they will specifically hybridise, to an adequate extent, with the target sequence.

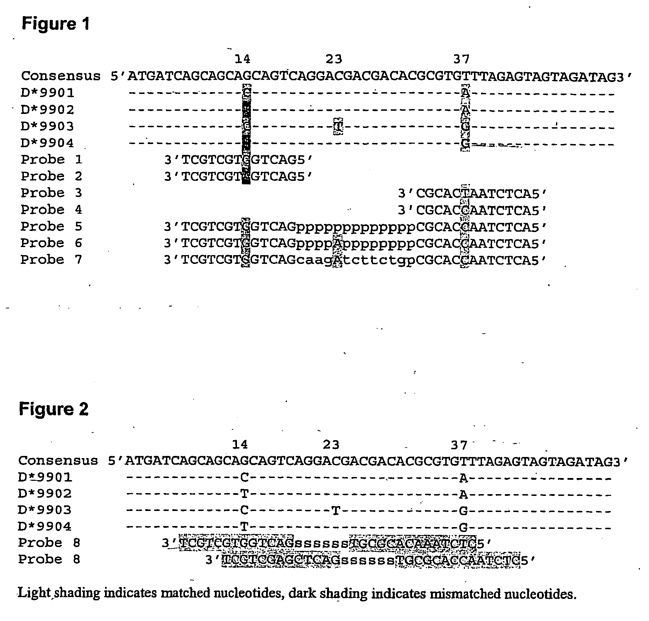

The probe of the invention has a particular advantage over prior art probes in the event that there are a number of polymorphic sequences, one or more of which represent the target sequence, in the sample to be probed. A hypothetical illustration of this advantage is illustrated in Figure 1. Referring to Figure 1, SSO probes 1-4 (of the prior art type) could be designed to descriminate between the alleles D*9901-*9904 using polymorphisms at positions 14 and 37. If a locus specific amplification was used and tested with the four probes and hybridized to a locus specific PCR-amplified DNA sample one possible result is that all the probes might be positive. If so, it would not be possible to determine which pair of alleles were present in the sample as D*9901 + D*9904 is the same as D*9902 + D*9903. However, when the two polymorphisms at positions 14 and 37 are linked (in *cis)* by a single probe (according to the invention), the probe is specific for the two non-contiguous sequence motifs. In Figure 1 probe 5 (according to the invention) is specific for the D*9903 allele.

The phosphoroamidite bridge in probe 5 (phosphoramidites in bridge indicated by p) is required to maintain pairwise binding between the probe and the target DNA sequence. Phosphoroamidites are unable to pair-base with target DNA strands and thus do not influence hybridisation temperature. If a probe (of the prior art type) were designed with standard matching nucleotides the probe's hybridisation temperature would be increased above the normal design constraints within an SSO typing system and the probe specificity would be compromised.

Still referring to Figure 1, probe 6 illustrates a further embodiment of the invention whereby a polymorphism on D*9903 allele is utilised to improve probe specificity by use of a conventional matched nucleotide in the correct spatial orientation in the spacer region of the probe. Probe 7 illustrates a further embodiment of the invention in which the spacer comprises (with one exception corresponding to the aforementioned polymorphism) mismatched nucleotides.

Certain prior art probes are known to contain inert spacers (as described in US-A-2002/0042077, for example). However, the spacers in these prior art probes are not selected (lengthwise) with regard to the target sequence to be effective for maintaining correct spatial orientation, and thus pairwise binding on hybridization, of the non-contiguous regions of the probe with *cis*-located regions on a target sequence. Figure 2 illustrates that such prior art probes that contain spacers composed of non-complementary sequences, e.g. incorrect length of spacer, may not provide an adequate or correct pair-wise binding signal due to improper binding. In this example probe 8 has an incorrect spacer length that does not allow correct pairwise binding of the probe hybridization regions to the target nucleic acid regions. In the first instance the left hand arm of the probe hybridizes to the target region but does not allow correct hybridization of the right-hand arm. The situation is reversed in the second example of probe 8 binding. Partial hybridization of probe to incorrect regions of DNA will result in both false positive and false negative results. These examples iterate the problems associated with prior art bridged probes.

The increased specificity owing to the ability of the probe of the invention to hybridise two or more non-contiguous regions of the target sequence reduces the number of alleles which can be detected by that probe and thus provides improved resolution over conventional SSO probing or any other technique involving oligonucleotide probes hybridizing to polymorphic target DNA sequences such as CDNA library screening or detection of RNA polymorphisms. The invention provides for *cis* (in phase) detection of spatially distinct target nucleic acid sequences with single diagnostic complementary DNA probes. The word *cis* refers to sequences that are in series on an allele, for example. The phrase 'spatially distinct' refers to discrete sequences that are located at different points along a gene or genome.

The probe preferably comprises first and second regions that are capable of hybridising to first and second non-contiguous regions on the target nucleic acid sequence respectively. The regions of the probe that are capable of hybridising to the target nucleic acid sequence may comprise at least one nucleotide complementary to at least one nucleotide on the target nucleic acid sequence. The preferred range of the hybridizing regions is between 1-20 complementary nucleotides.

The probe may comprise the equivalent of 1 to 300 nucleic acid bases in total length. Preferably the probe comprises the equivalent to 1-200 nucleic acid bases. More preferably still, the probe comprises the equivalent to 30-100 nucleic acid bases.

Preferably, the probe is capable of hybridising to HLA alleles, but may include any polymorphic loci where there are two or more polymorphic regions on one or more alleles within said loci.

The probe of the invention may correspond to a formula as follows: 5'-Hybl-(1-300sp)-Hyb2,
e.g. 5'amino-cacgttatectcctgg(13P)tgtccaggttccgca;
   5'amino-cgcacgttatcctcctg(14P)tgtccaggttecgca; or
   5'amino-cgcacgttatcctcct(15P)tgtccaggttccgca,
   5'-Hybl-(1-150sp)-Hyb2-(1-150sp)-Hyb3,
e.g. 5'amino-cacgttatcctcctgg(13P)tgtccaggttccgca(17P)tttgatacgacgatagcga, 5'-Hyb1-[(1-Nsp)-(1-10 nucleotide)-(1-Nsp)-)-(1-10 nucleotide)-(1-Nsp)]-Hyb2,
e.g. 5'amino-cacgttatcctcctgg(4xP)g(2xP)a(1xP)ta(2xP)tgtccaggttccgca,
wherein 'sp' denotes a spacer molecule, such as a mismatched nucleotide or a phosphoramidite, for example, P = phosphoramidite (by way of an example), Hyb1 = first hybridizing nucleotide region comprising, for example, 1-30 nucleotide bases, Hyb2 = second hybridizing nucleotide region comprising, for example, 1-30 nucleotide bases and Hyb3 = third hybridizing nucleotide region comprising, for example, 1-30 nucleotide bases.

In accordance with a further aspect of the present invention there is provided a method for detecting specifically two or more *cis*-located target nucleic acid sequence in a sample comprising contacting said sample with at least one probe as described herein above and determining whether any probe/target nucleic acid sequence hybrid is formed.

In accordance with a further aspect of the present invention there is provided a method for detecting specifically non-contiguous *cis* located polymorphic target nucleic acid sequences in a nucleic acid sample comprising contacting the sample with at least one probe as described hereinabove and determining whether any probe/target nucleic acid sequence hybrid is formed.

Preferably the methods described hereinabove comprise the pre-step of amplifying the nucleic acid sample using a technique known in the art such as, for example, polymerase chain reaction (PCR) (Saiki RK et al Science. 1985 Dec 20;230(4732):1350-4).

Preferably, the method of the invention comprises contacting the probes and sample nucleic acid under hybridising conditions. The conditions may be those used in standard SSO techniques.

In accordance with a further aspect of the present invention there is provided an apparatus for detecting a target nucleic acid sequence in a sample, the apparatus comprising a sample application zone and at least one probe as described hereinabove. It is a preferred feature of the present invention that the apparatus for detecting a target nucleic acid sequence in a sample may be used in a biological assay. Preferably, the biological assay is used for the detection of one or more sequences in a sample. More preferably, the biological assay is used for to detect polymorphisms in human leukocyte antigens (HLA).

In accordance with another aspect of the present invention, there is provided a method of diagnosis comprising contacting a probe as herein described, designed to hybridise to an allele or number of alleles and/or mutations, with a sample from an individual and detecting the presence or absence of a resulting hybrid in order to determine the genotype of the individual. This method of diagnosis can be used to assess the precise genetic nature of an individual's condition or disease or to establish the pre-disposition of an individual to a particular condition or disease. Furthermore, the method of diagnosis may also be used to assess genotypic information on an individual. Preferably, the method of diagnosis is used to establish the status of the alleles of the human leukocyte antigens in an individual. It will be apparent to one skilled in the art that the method may be directed towards investigating other polymorphic genes.

In order that the method of the invention may be clearly understood and readily put into effect, a protocol for its operation will now be described. This protocol was used, unless otherwise indicated, in the Examples below.

The probes are conjugated to latex beads via a 5'-amino linker that binds the probe to carboxylate moieties on the bead surface. Briefly, the conjugation process requires incubation of carboxylate beads with amino-labelled oligonucleotides in the presence of 2M 1-Ethyl-3-(3-dimethylaminopropyl) carbodiimide hydrochloride (EDC), and 50mM N-hydroxysulfosuccinimide (NHS). Un-conjugated probe is subsequently washed away and the beads are resuspended in water and dotted onto nylon membrane (Cuno 0.8 micron) where they are allowed to dry onto the membrane.

Prior to the hybridization assay the sample DNA is PCR amplified: The DNA-containing specimen and reagent mixture are heated to 95°C to separate the double-stranded DNA and expose the target sequences to the primers. As the mixture cools, the biotinylated primers anneal to their targets. The thermostable recombinant *Thermus aquaticus* (Taq) DNA polymerase in the presence of excess deoxynucleoside triphosphates (dNTPs), including deoxyadenosine, deoxyguanosine, deoxycytidine and deoxythymidine or deoxyuridine (deoxyuridine for RNA), extends the annealed primers along the target templates to produce a biotinylated DNA sequence termed an amplicon. This process is repeated for a number of cycles, each cycle effectively doubling the amount of target DNA. For this test, an adequate number of cycles has been determined to be 35, theoretically yielding more than a billion-fold amplification.

After the PCR amplification process, the amplicons are chemically denatured (using a solution containing 3% EDTA, 1.6% sodium hydroxide) to form single strands which are then added to a well of a typing tray that contains the nylon membrane with the immobilized, sequence-specific, oligonucleotide probes. The biotin-labeled amplicons then bind (hybridise) to the sequence-specific probes and thus are "captured" onto the membrane strip. The stringent conditions for hybridisation of the amplicons to the probes ensure the specificity of the reaction.

After a stringent wash of the membrane strip to remove unbound material, a streptavidin-horseradish peroxidase (SA-HRP) conjugate is added to the well of the tray. The streptavidin binds to the biotin-labeled amplicons captured by the membrane-bound probe. After washing off unbound conjugate, the bound SA-HRP conjugate is reacted with hydrogen peroxide (H₂O₂) and tetramethylbenzidine (TMB) to form a colour complex. The reaction is stopped by several water washes. The hybridization stages may be performed manually or may be performed automatically using the Dynal AutoRELI^{™} 48 automated strip development apparatus (available from Dynal Biotech Ltd).

The developed strips can then be scanned using a flat-bed optical scanner and the results can be interpreted manually or by using an analysis program such as Dynal's Pattern Matching Program (PMP).

The present invention will now be described, by way of example only, with reference to the following Figures and Examples, in which:
Figure 1 illustrates the principle of bridged probes, discussed above;
Figure 2 illustrates the problem associated with probes that do not contain a correct length bridging sequence to maintain pair-wise binding of hybridizing regions, discussed above.
Figure 3 illustrates probes according to the invention aligned with selected target sequences; and
Figure 4 illustrates the results of probes according to the invention hybridised with nucleic acid samples;

### EXAMPLES

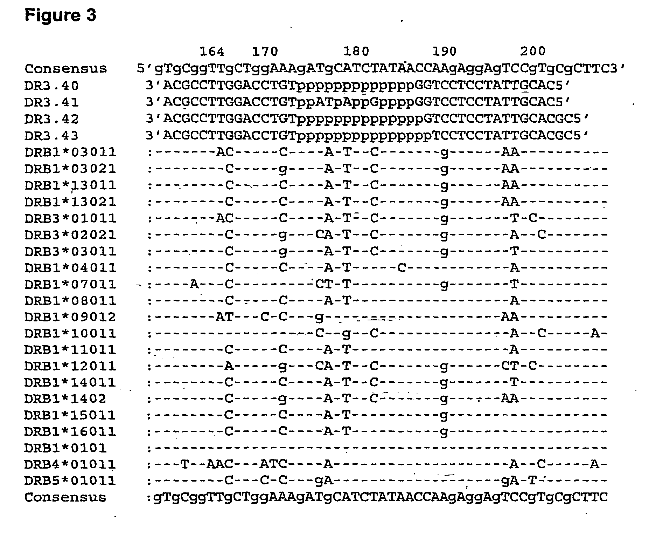

These experiments were conducted in order to investigate the hybridisation of probes specific for certain alleles (DRB1*0301/5 etc). Referring now to Figure 3, various test probes (see below) were designed for a combination of 164A-165C-172C combined with 190G-197A-198A polymorphisms that together are unique for DRB1 *0301/5/6/8/10-13/15/15/18-20 alleles when used on target DNA amplified with DRB 1 locus specific primers. The probes DR3.40-43 are bridged. Phosphoramidites indicated by `p'. The probes were designed to give positive reactions only when both arms of the bridged probes are matched for an individual allele: the so called 'intersection specificity'. These probes are designed to have an intersection specificity of DRB1*03011/*03012, *03051/03052, *0308/10/11-16/18-20, *1327/41 & DRB3*0108. Cross-reactivity of the left arm might be expected (for example) with DRB3*0101 alleles whilst cross-reactivity of the right arm might be expected with DRB 1*1301 or DRB1*1302 alleles. DNA samples containing these potentially false-positive alleles were tested along with true positive DNA samples for DRB1*0301. The sequences of each probe is outlined in the table below.

| Identity | Sequence |
|---|---|
| DR3.40 | 5'amino-cacgttatcctcctgg(13xp)tgtccaggttccgca |
| DR3.41 | 5'amino-cacgttatcctcctgg(4xp)g(2xp)a(1xp)ta(2xp)tgtccaggttccgca |
| DR3.42 | 5'amino-cgcacgttatcctcctg(14xp)tgtccaggttccgca |
| DR3.43 | 5'amino-cgcacgttatcctcct(15xp)tgtccaggttccgca |

### Probe specificity:

The probe region focusing on ~ position 164 (left arm) matches the alleles DRB 1*03011/2, DRB 1*0304, DRB 1*03051/2, DRB 1*0306, DRB 1*0308, DRB1*0309, DRB1*0310, DRB1*0311, DRB1*0312, DRB1*0313, DRB1*0314, DRB1*0315, DRB1*0316, DRB1*0318, DRB1*0319, DRB1*0320, DRB1*1327, DRB1*1341, DRB3*01011, DRB3*01012, DRB3*0101202, DRB3*01013, DRB3*01014, DRB3*0102, DRB3*0104, DRB3*0106, DRB3*0107, DRB3*0108, DRB3*0110

The second region focusing on ~ position 196 (right arm) matches the alleles DRB1*1608, DRB1*03011, DRB1*03012, DRB1*03021, DRB1*03022, DRB1*0303, DRB1*03051, DRB1*03052, DRB1*0306, DRB1*0307, DRB1*0308, DRB1*0310, DRB1*0311, DRB1*0312, DRB1*0313, DRB1*0314, DRB1*0315, DRB1*0316, DRB1*0318, DRB1*0319, DRB1*0320; DRB1*1109; DRB1*1116, DRB1*1120; DRB1*1140, DRB1*13011, DRB1*13012, DRB1*13021, DRB1*13022, DRB1*1305, DRB1 *1306, DRB 1*1309, DRB 1*1310, DRB1*1315, DRB 1*1316, DRB1*1318, DRB1*1320, DRB1*1326, DRB1*1327, DRB1*1328, DRB1*1329, DRB1*1331, DRB1*1332, DRB1*1335, DRB1*1336, DRB1*1339, DRB1*1340, DRB1*1341, DRB1*1342, DRB1*1343, DRB1*1402, DRB1*1403, DRB1*1406, DRB1*1409, DRB1*1412, DRB1*1413, DRB1*1417, DRB1*1418, DRB1*1419, DRB1*1421, DRB1*1424, DRB1*1427, DRB1*1429, DRB1*1430, DRB1*1433, DRB3*0108

### Intersection specificity:

Combining both halves of the bridged probes gave an overall specificity for the probe of the following alleles. DRB1*03011/*03012,*03051/03052, 0308/10/11-16/18-20, 1327, 1341 & 3*0108.

Probes were conjugated to 0.1micron Polysciences latex particles and were subsequently dotted onto Cuno 0.8 micron nylon membrane and allowed to air dry at room temperature. DNA samples were amplified by PCR using generic primers for DRB1/3/4/5 (CRX28 5'-biotin-CCGGATCCTTCGTGTCCCCACAGCACG, AB60 5'-biotin-CCGAATTCCGCTGCACTGTGAAGCTCTC) and alternatively by DRB 1 (primers D6 plus DAS6 or D6 plus DAS 1 depending on cell line genotype) or DRB3 specific primers D9V plus DAS1 or D9V-plus DAS6, again depending on the phenotype). Primers D6, D9V, DAS 1 and DAS6 anneal to their targets at positions 15-38, 12-31, 257-278 and 257-278 respectively.

DNA from the following samples were amplified

| **Position on Figure** | **Cell line** | **DRB1** | **DRB3** |
|---|---|---|---|
| **4** | **identity** | | |
| 1 | 30 | *0302 | *0101 |
| 2 | 7 | *0301 | *0202 |
| 3 | 104 | *0301 | *0101 |
| 4 | 41 | *1302 | *0301 |
| 5 | 37 | *1301 | *0101 |
| 6 | 21 | *1402 | *0101 |
| 7 | 55 | *1401 | *0202 |
| 8 | 16 | *1101 | *0202 |
| 9 | 17 | *1102 | *0202 |
| 10 | 8 | *0401, | |
| | | *1602 | |

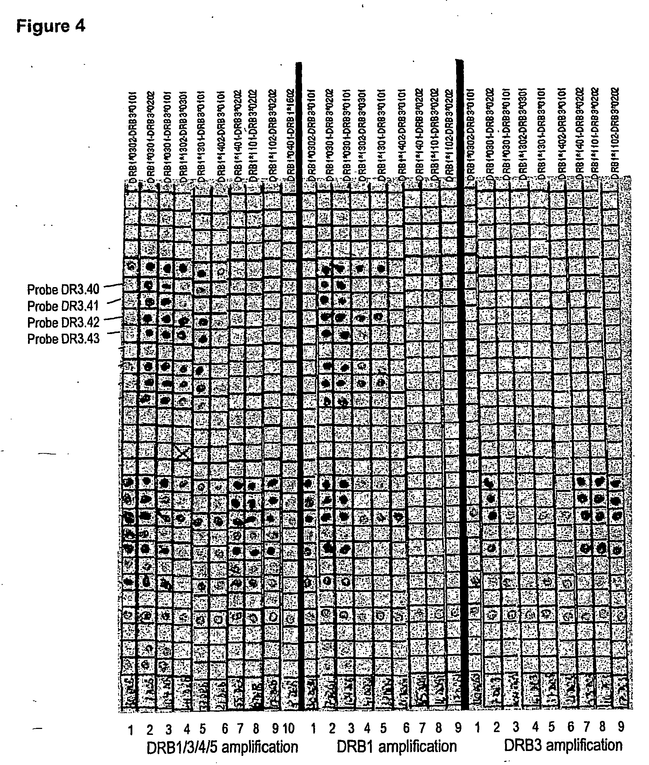

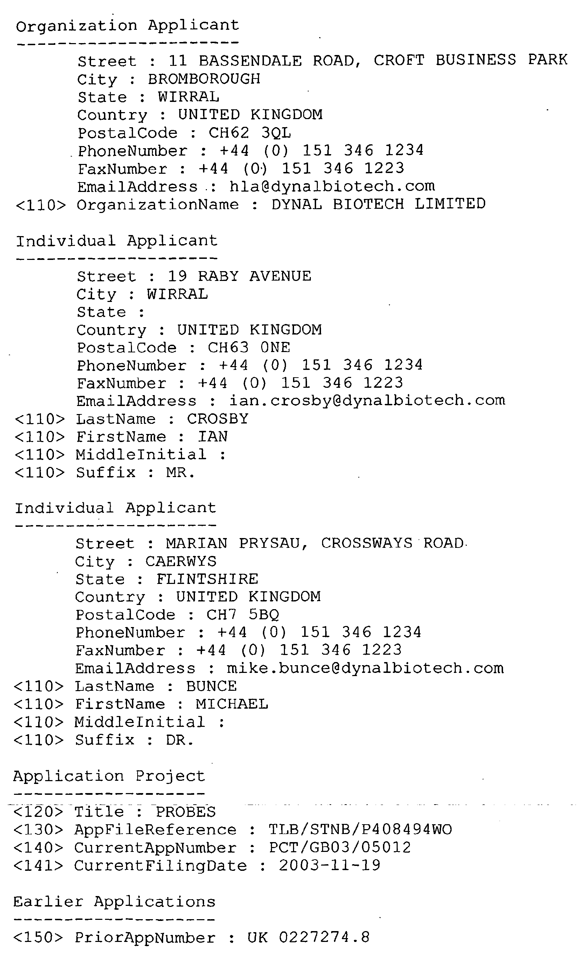

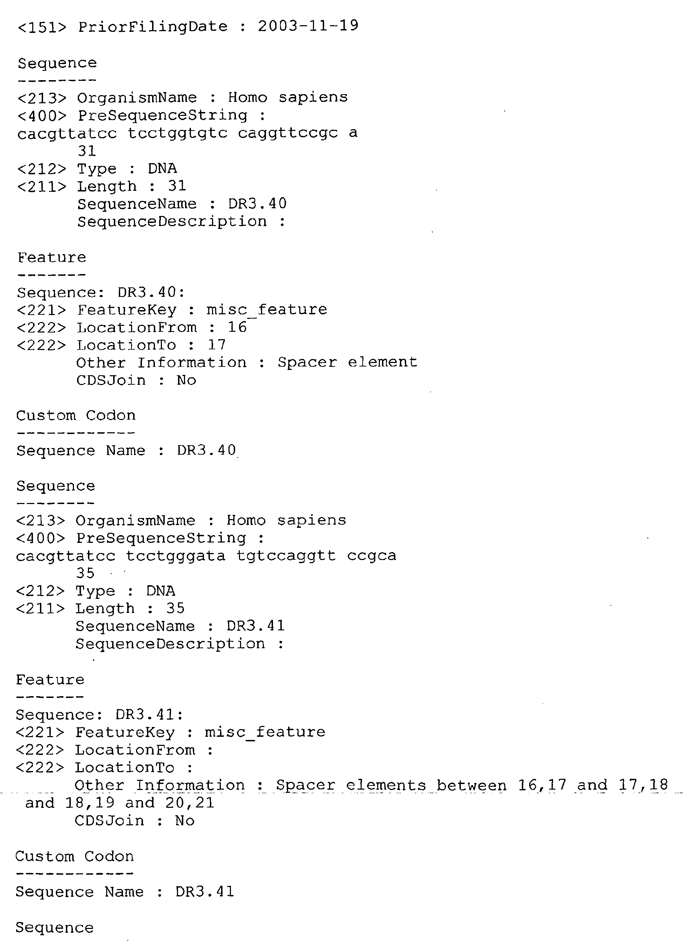

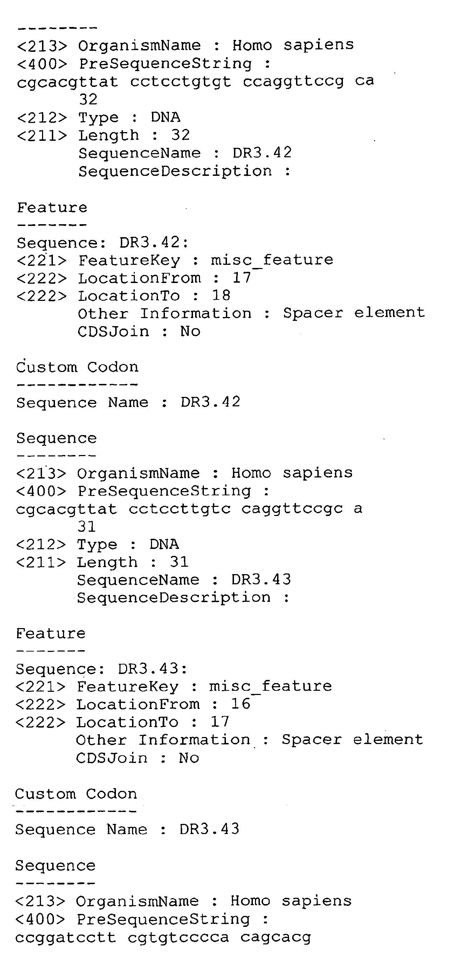

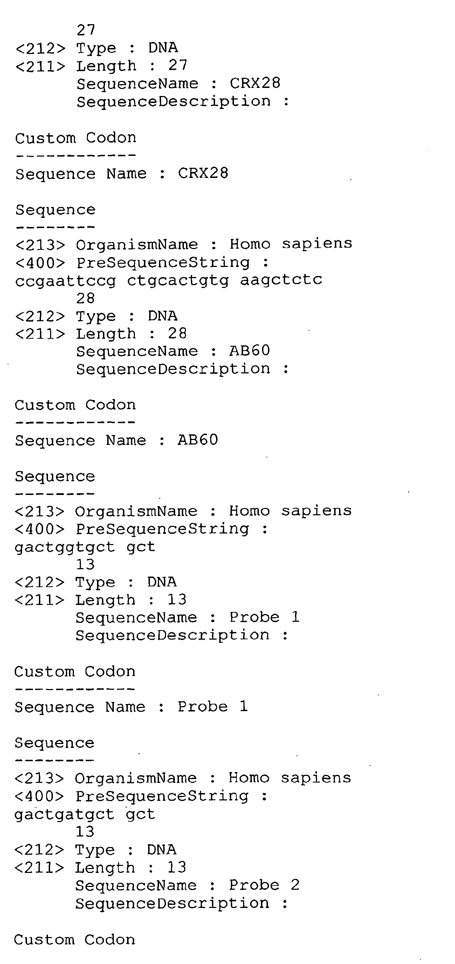

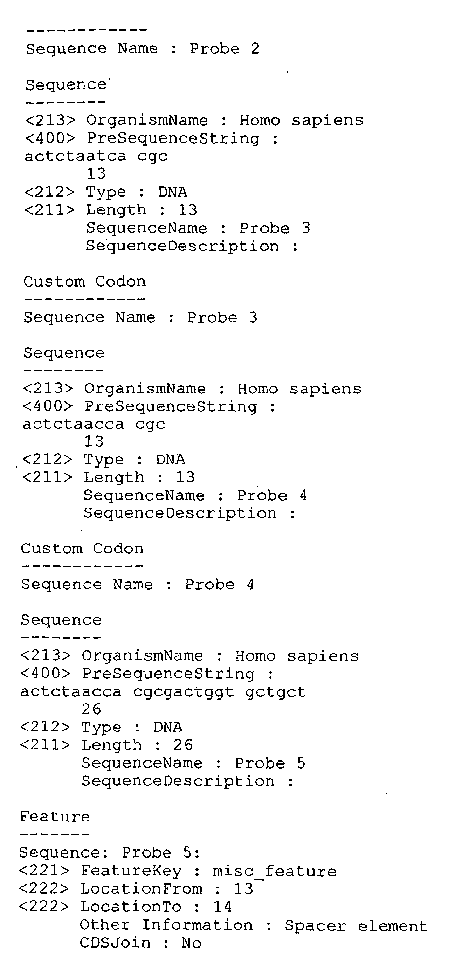

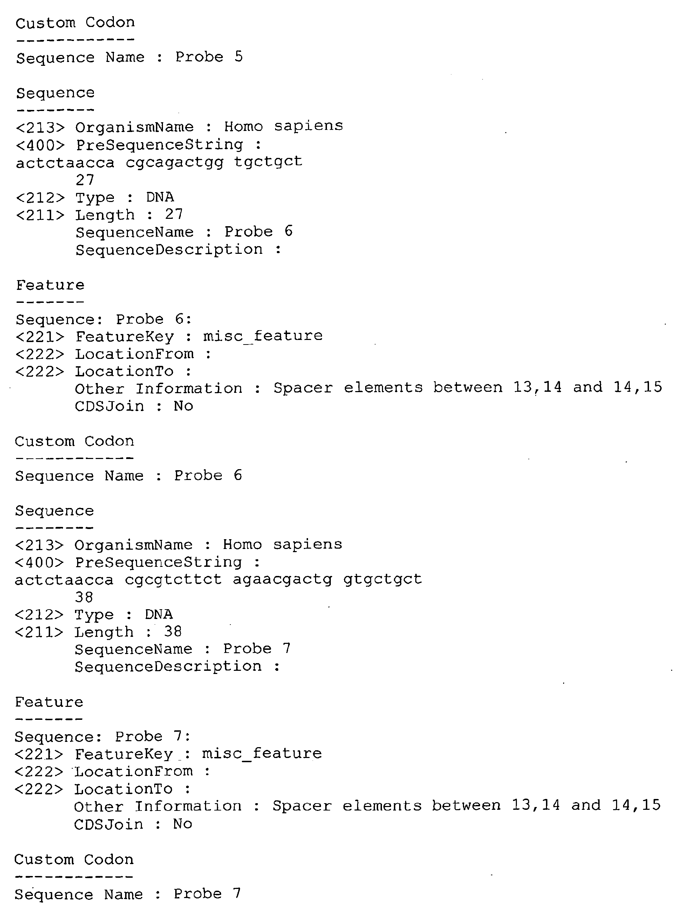

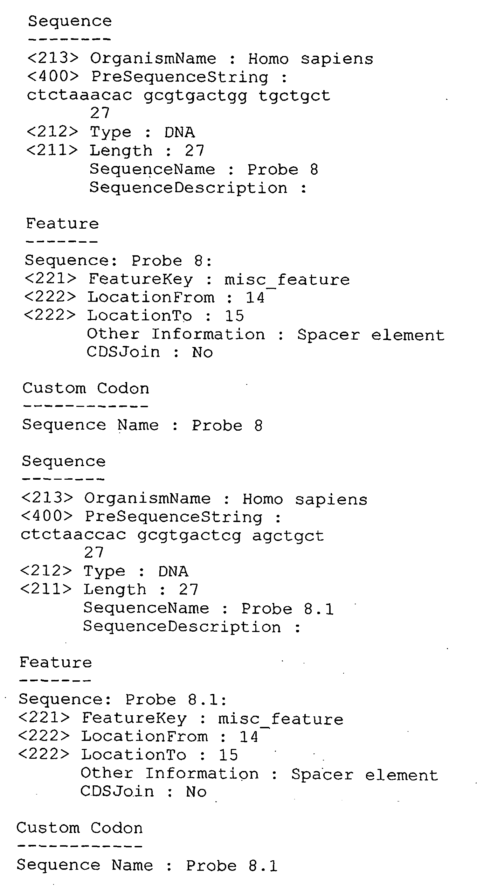

Hybridisation was carried out under the standard protocol described above. The results are shown in Figure 4 and discussed below.

The results show that probe DR3.41 is specific for DRB 1*0301 without cross-reactivity to the closely related DRB 1*1301, *1302 or DRB3*0101 alleles. The best specificity is seen with DRB 1 amplification as amplification with generic primers (DRB 1/3/4/5) results in some cross-reactivity with DRB1*13. Probe DR3.41 is an example of a bridged probe with additional nucleoside matches within the probe bridge.

Probe DR3.40 is an example of a probe with the bridge entirely constructed from phosphoramidites, Probe DR3.40 is most similar to probe DR3.41 but does not have the intervening nucleotides in the bridge, rather the bridge is made up entirely of phosphoramidites. This example is also specific for DRB1 *0301 with no cross-reactivity with the closely related DRB1*13 alleles or DRB3*0101 alleles when DRB1-specific amplification is used. If generic amplification with DRB 1/3/4/5 primers is used there is some residual cross-reactivity observed with DRB 1*13 alleles.

## Claims

1. A nucleic acid probe for detecting a target nucleic acid sequence comprising two or more *cis*-located nucleic acid regions, the probe comprising two or more non-contiguous nucleic acid regions capable of hybridising with respective cis-located nucleic acid regions on the target sequence, the non-contiguous regions being separated by one or more spacer regions comprising at least predominantly material which will not hybridise with the target sequence in the region between the cis-located regions and having a length selected with regard to the target sequence effectively to maintain correct spatial orientation of the non-contiguous hybridising regions on the probe with the cis-located regions on the target sequence such that base-base pairwise hybridisation therebetween can be effected in use of the probe wherein at least one of the one or more spacer regions comprise(s) one or more nucleic acids matched for hybridisation with corresponding one or more nucleic acids in the target sequence in the region between the cis-located regions and/or wherein the spacer region separating the two or more non-contiguous nucleic acids comprises a sequence of molecules, each of which are the same, or a similar size to a nucleic acid base, and wherein at least one of the one or more spacer regions comprise(s) abasic phosphoramidite nitose molecules.

2. A nucleic acid probe according to claim 1 wherein the length of the spacer region is selected to be substantially equal in length to the length of that region of the target sequence which lies between the *cis-*located regions.

3. A nucleic acid probe according to either one of claims 1 or 2 wherein at least one of the one or more spacer regions comprise(s) a nucleic acid sequence mismatched with respect to the target sequence in the region between the cis-located regions.

4. A nucleic acid probe according to any one of claims 1 to 3 wherein the probe comprises the equivalent to 1 to 300 nucleotide bases.

5. A nucleic acid probe according to any one of claims 1 to 4 wherein the target nucleic acid sequence comprises one or more alleles of a gene.

6. A nucleic acid probe according to any one of claims 1 to 5 wherein the target nucleic acid sequence comprises the human leukocyte antigen gene (HLA).

7. A nucleic acid probe according to any one of claims 1 to 5 wherein the target nucleic acid sequence comprises the heamochromatosis gene (HFE).

8. A nucleic acid probe according to any one of claims 1 to 5 wherein the target nucleic acid sequence comprises the thiopurine methyl transferase gene (TPMT).

9. A nucleic acid probe according to any one of claims 1 to 5 wherein the target nucleic acid sequence comprises the tumour necrosis factor gene (TNF).

10. A nucleic acid probe according to any one of claims 1 to 9 wherein the probe comprises any one or more sequences selected from the following group:
5`-Hyb1-(1-300sp)-Hyb2,
e.g. 5'amino-cacgttatcctcctgg(13P)tgtccaggttccgca;
5'amino-cgcacgttatcctcctg(14P)tgtccaggttccgca; or
5'amino-cgcacgttatcctcct(15P)tgtccaggttccgca.
5'-Hyb1-(1-15dsp)-Hyb2-(1-150sp)-Hyb3,
e.g. 5'amino-cacgttatcctcctgg(13P)tgtccaggttcegca(17P)tttgatacgacgatagcga, 5'-Hybl-[(1-Nsp)-(1-10 nucleotide)-(1-Nsp)-)-(1-10 nucleotide)-(1-Nsp)]-Hyb2,
e.g. 5'amino-cacgttatcctcctgg(4xP)g(2xP)a(1xP)ta(2xP)tgtccaggttccgca,
wherein 'sp' denotes a spacer molecule, such as a mismatched nucleotide or a phosphoramidite, for example, P = phosphoramidite (by way of an example), Hyb1 = first hybridizing nucleotide region comprising, for example, 1-30 nucleotide bases, Hyb2 = second hybridizing nucleotide region comprising, for example, 1-30 nucleotide bases and Hyb3 = third hybridizing nucleotide region comprising, for example, 1-30 nucleotide bases.

11. A probe kit comprising first and second nucleic acid probes according to any one of claims 1 to 10, the first probe differing from the second by at least one nucleic acid base in at least one of the non-contiguous regions such that the first probe is capable of hybridizing with a first target sequence and the second probe is capable of hybridizing with a second, polymorphic, target sequence.

12. A method for detecting a target nucleic acid sequence in a sample comprising contacting said sample with at least one nucleic acid probe according to any one of claims 1 to 10 and determining the presence of any hybridized material.

13. A method according to claim 12 comprising a pre-step of amplifying the nucleic acid sample using the polymerase chain reaction (PCR).

14. A method according to claim 12 or claim 13 comprising a step of contacting the probe and sample nucleic acid under hybridising conditions.

15. A method according to any one of claims 12 to 14 comprising a step of contacting the probes and sample nucleic under a standard sequence-specific oligonucleotide (SSO) protocol.

16. An apparatus for detecting a target nucleic acid sequence in a sample, wherein the apparatus comprises a sample application zone and at least one probe according to any one of claims 1 to 10.

17. An apparatus according to claim 16, wherein the apparatus is used in a biological assay.

18. An apparatus according to claim 17, wherein the biological assay is used for the detection of one or more sequences in a sample.

19. An apparatus according to any one of claims 16 to 18 wherein the biological assay is used to detect polymorphisms in human leukocyte antigens (HLA).

20. A method for detecting a target nucleic acid sequence in a sample comprising contacting a sample with at least one probe according to claims 1 to 10, and determining whether any at least one probe/target nucleic acid sequence hybrid is formed

21. A method of diagnosis comprising contacting a probe according to claims 1 to 10, designed to hybridise to an allele or number of alleles and/or mutations with a sample from an individual in order to determine the genotype of the individual.

22. A method of diagnosis according to claim 21, wherein method of diagnosis is used to establish the status of the alleles of human leukocyte antigens in an individual.

## Patentansprüche

1. Eine Nukleinsäuresonde für das Nachweisen einer Zielnukleinsäuresequenz umfassend zwei oder mehrere cis-ständige Bereiche der Nukleinsäure, wobei die Sonde zwei oder mehrere nicht angrenzende Bereiche der Nukleinsäure, die mit entsprechenden cis-ständigen Bereichen der Nukleinsäure auf der Zielsequenz hybridisieren können, wobei die nicht angrenzenden Bereiche von einer oder mehreren Spacer Regionen getrennt werden, die zumindest vorwiegend Materialien umfassen, die nicht mit der Zielsequenz in dem Bereich zwischen den cis-ständigen Bereichen hybridisieren und die eine Länge aufweisen, die mit Bezug auf die Zielsequenz ausgewählt wurde, um in wirksamer Weise die korrekte räumliche Ausrichtung der nicht angrenzenden hybridisierenden Bereiche auf der Sonde zu den cis-ständigen Bereichen auf der Zielsequenz beizubehalten, so dass dazwischen unter Verwendung der Sonde eine paarweise Base zu Base Hybridisierung ausgeführt werden kann, wobei wenigstens eine der einen oder mehreren Spacer Regionen eine oder mehrere Nukleinsäure(n) umfasst (umfassen) die mit der Zielsequenz in dem Bereich zwischen den cis-ständigen Bereichen mit der (den) entsprechenden einen oder mehreren Nukleinsäure(n) hybridisieren können und/ oder worin die Spacer Region, welche die zwei oder mehreren nicht angrenzende(n) Nukleinsäuren trennt, eine Sequenz von Molekülen, von denen ein jedes die selbe oder eine ähnliche Größe wie eine Nukleinsäurebase aufweist und worin wenigstens eine der einen oder mehreren Spacer Regionen abasische Phosphoramidit-Ribose-Moleküle umfasst.

2. Eine Nukleinsäuresonde gemäß Anspruch 1, wobei die Länge der Spacer Region so ausgewählt wird, dass sie im Wesentlichen in ihrer Länge der Länge desjenigen Bereichs der Zielsequenz entspricht, der zwischen den cis-ständigen Bereichen liegt.

3. Eine Nukleinsäuresonde gemäß entweder Anspruch 1 oder Anspruch 2, wobei mindestens eine der einen oder mehreren Spacer Region(en) eine Nukleinsäuresequenz umfasst, die in Bezug auf die Zielsequenz in dem Bereich zwischen den cis-ständigen Bereichen fehlgepaart ist.

4. Eine Nukleinsäuresonde gemäß einem der Ansprüche 1 bis 3, wobei die Sonde das Äquivalent zu 1 bis 300 Nukleotidbasen umfasst.

5. Eine Nukleinsäuresonde gemäß einem der Ansprüche 1 bis 4 wobei die Zielnukleinsäuresequenz ein oder mehrere Allel(e) eines Gens umfasst.

6. Eine Nukleinsäuresonde gemäß einem der Ansprüche 1 bis 5, wobei die Zielnukleinsäuresequenz das Gen für das Humane Leukozyten Antigen (HLA) umfasst .

7. Eine Nukleinsäuresonde gemäß einem der Ansprüche 1 bis 5, wobei die Zielnukleinsäuresequenz das Gen für Hämatochromatose (HFE) umfasst.

8. Eine Nukleinsäuresonde gemäß einem der Ansprüche 1 bis 5, wobei die Zielnukleinsäuresequenz das Gen für Thiopurinmethyltransferase (TPMT) umfasst.

9. Eine Nukleinsäuresonde gemäß einem der Ansprüche 1 bis 5, wobei die Zielnukleinsäuresequenz das Gen für Tumor-Nekrose-Faktor (TNF) umfasst.

10. Eine Nukleinsäuresonde gemäß einem der Ansprüche 1 bis 9, wobei die Sonde eine oder mehrere beliebige Sequenzen ausgewählt aus der folgenden Gruppe umfasst:
5'-Hybl- (1-300sp)-Hyb2,
z. B. 5'amino-cacgttatcctcctgg(13P)tgtccaggttccgca;
5'amino-cgcacgttatcctcctg(14P)tgtccaggttccgca; or
5'amino-cgcacgttatcctcct(15P)tgtccaggttccgca,
5'-Hybl-(1-150sp)-Hyb2-(1-150sp)-Hyb3,
z. B. 5'amino-cacgttatcctcctgg(13P)tgtccaggttccgca(17P)tttgatacgacgatagcga, 5'-Hyb1-[(1-Nsp)-(1-10 Nukleotid)-(1-Nsp)-)-(1-10 Nukleotid)-(1-Nsp)]-Hyb2,
z.B. 5'amino-cacgttatcctcctgg(4xP)g(2xP)a(1xP)ta(2xP)tgtccaggttccgca,
worin "sp" ein Spacer Molekül, wie zum Beispiel einen fehlgepaarten Nukleotid oder ein Phosphoramidit bezeichnet; P = Phosphoramidit (als Beispiel); Hyb1 = erster hybridisierender Bereich des Nukleotids, der zum Beispiel 1-30 Nukleotidbasen umfasst, Hyb2 = zweiter hybridisierender Bereich des Nukleotids, der zum Beispiel 1-30 Nukleotidbasen umfasst und Hyb3 = dritter hybridisierender Bereich des Nukleotids, der zum Beispiel 1-30 Nukleotidbasen umfasst.

11. Ein Sondensatz umfassend erste und zweite Nukleinsäuresonden gemäß einem der Ansprüche 1 bis 10, wobei sich die erste Sonde von der zweiten Sonde durch mindestens eine Nukleinsäurebase in mindestens einem der nicht angrenzenden Bereiche unterscheidet, so dass die erste Sonde mit einer ersten Zielsequenz hybridisieren kann und die zweite Sonde mit einer zweiten, polymorphen Zielsequenz hybridisieren kann.

12. Ein Verfahren für das Nachweisen einer Nukleinsäuresequenz in einer Probe welches umfasst, dass diese Probe mit mindestens einer Nukleinsäuresonde gemäß einem der Ansprüche 1 bis 10 in Kontakt gebracht wird und dass bestimmt wird, ob beliebiges hybridisiertes Material vorliegt.

13. Ein Verfahren gemäß Anspruch 12, welches einen vorbereitenden Schritt umfasst, in dem die Nukleinsäuresonde unter Verwendung der Polymerase-Kettenreaktion (PCR) amplifiziert wird.

14. Ein Verfahren gemäß Anspruch 12 oder Anspruch 13, das eine Schritt umfasst, in dem die Sonde und die Nukleinsäurenprobe unter Hybridisierungsbedingungen in Kontakt gebracht werden.

15. Ein Verfahren gemäß einem beliebigen von Anspruch 12 bis 14, das eine Schritt umfasst, in dem die Sonde und die Nukleinsäurenprobe gemäß einem Sequenzspezifischen Oligonukleotid (SSO) Standardprotokoll in Kontakt gebracht werden.

16. Ein Gerät für das Nachweisen eine Zielnukleinsäuresequenz in einer Probe wobei der Apparat einen Bereich für das Aufbringen der Probe und mindestens eine Sonde gemäß einem der Ansprüche 1 bis 10 umfasst.

17. Ein Gerät gemäß Anspruch 16, wobei der Apparat in einem biologischen Assay verwendet wird.

18. Ein Gerät gemäß Anspruch 17, wobei biologische Assay für den Nachweis einer oder mehrerer Sequenzen in einer Probe verwendet wird.

19. Ein Gerät gemäß einem beliebigen der Ansprüche 16 bis 18, wobei der biologische Assay verwendet wird, um Polymorphismen im Humanen Leukozyten Antigen (HLA) nachzuweisen.

20. Ein Verfahren für das Nachweisen einer Zielnukleinsäuresequenz in einer Probe, das umfasst, dass diese Probe mit mindestens einer Sonde gemäß einem der Ansprüche 1 bis 12 in Kontakt gebracht wird und dass bestimmt wird, ob zumindest ein Sonden-/ Zielnukleinsäuresequenz-Hybrid gebildet wird.

21. Ein Diagnoseverfahren, welches umfasst, dass eine Sonde gemäß den Ansprüchen 1 bis 10, die so ausgeführt ist dass sie an ein Allel oder eine Mehrzahl von Allelen und/ oder Mutationen hybridisiert, mit einer Probe von einem Individuum in Kontakt gebracht wird, um den Genotyp des Individuums zu bestimmen.

22. Ein Diagnoseverfahren gemäß Anspruch 21, wobei das Diagnoseverfahren verwendet wird, um den Status der Allele von Humanen Leukozyten Antigenen in einem Individuum festzustellen.

## Revendications

1. - Sonde d'acide nucléique pour détecter une séquence d'acide nucléique cible comprenant deux régions d'acide nucléique situées en *cis* ou davantage, la sonde comprenant deux régions d'acide nucléique non contiguës ou davantage, capables de s'hybrider avec des régions d'acide nucléique situées en *cis* respectives sur la séquence cible, les régions non contiguës étant séparées par une ou plusieurs régions espaceurs comprenant au moins de façon prédominante un matériel qui ne s'hybridera pas à la séquence cible dans la région entre les régions situées en *cis* et ayant une longueur choisie par rapport à la séquence cible pour maintenir de façon effective une orientation spatiale correcte des régions hybridantes non contiguës sur la sonde avec 1 es régions situées en *cis* sur la séquence cible de telle sorte qu'une hybridations par paires base-base entre celles-ci peut être effectuée à l'aide de la sonde, au moins l'une parmi la ou les différentes régions espaceurs comprenant un ou plusieurs acides nucléiques appariés pour l'hybridation avec un ou plusieurs acides nucléiques correspondants dans la séquence cible dans la région entre les régions situées en cis et/ou la région espaceur séparant les deux acides nucléiques non-contigus ou davantage comprenant une séquence de molécules, dont chacune est d'une dimension identique ou analogue à une base d'acide nucléique, et au moins l'une parmi la ou les différentes régions espaceur s comprend des molécules de phosphoramidite ribose abasiques.

2. - Sonde d 'acide nucléique selon la revendication 1, dans laquelle la longueur de la région espaceur est choisie pour être sensiblement égale en longueur à la longueur de cette région de la séquence cible qui s'étend entre les régions situées en *cis.*

3. - Sonde d'acide nucléique selon l'une ou l'autre des revendications 1 ou 2, dans laquelle au moins l'une de la ou des différentes régions espaceurs comprend une séquence d'acide nucléique mésappariée par rapport à la séquence cible dans la région entre les régions situées en *cis*.

4. - Sonde d'acide nucléique selon l'une quelconque des revendications 1 à 3, dans laquelle la sonde comprend l'équivalent de 1 à 300 bases nucléotidiques.

5. - Sonde d'acide nucléique selon l'une quelconque des revendications 1 à 4, dans laquelle la séquence d'acide nucléique cible comprend un ou plusieurs allèles d'un gène.

6. - Sonde d'acide nucléique selon l'une quelconque des revendications 1 à 5, dans laquelle la séquence d'acide nucléique cible comprend le gène de l'antigène leucocytaire humain (HLA).

7. - Sonde d'acide nucléique selon l'une quelconque des revendications 1 à 5, dans laquelle la séquence d'acide nucléique cible comprend le gène de l'hémochromatose (HFE).

8. - Sonde d'acide nucléique selon l'une quelconque des revendi cations 1 à 5, dans laquelle la séquence d'acide nucléique cible comprend le gène de la thiopurine méthyl transférase (TPMT).

9. - Sonde d'acide nucléique selon l'une quelconque des revendications 1 à 5, dans laquelle la séquence d'acide nucléique cible comprend le gène du facteur nécrosant des tumeurs (TNF).

10. - Sonde d'acide nucléique selon l'une quelconque des revendications 1 à 9, dans laquelle la sonde comprend une ou plusieurs séquences quelconques choisies dans le groupe suivant :
5'-Hybl-(1-300sp)-Hyb2,
par exemple :
5'amino-cacgttatcctcctgg(13P)tgtccaggttccgca;
5'amino-cgcacgttatcctcctg(14P)tgtccaggttccgca,
ou
**5'amino-cgcacgttatcctcct(15P)tgtccaggttccgca**,
5'-Hybl-(1-150sp)-Hyb2-(1-150sp)-Hyb3,
par exemple .
5'amino-cacgttaicctcctgg(13P)tgtccaggttccgca(17P)tttgatacgacgatagcga,
5'-Hybl-[(1-Nsp)-(1-10 nucléotides)-(1-Nsp)-(1-10 nucléotides)-(1-Nsp)]-Hyb2,
par exemple :
5'amino-cacgttatcctcctgg(4xP)g(2xP)a(1xP)ta(2xP)tgtccaggttccgca,
où « sp » désigne une molécule espaceur, telle qu'un nucléotide mésapparié ou un phosphoramidite, par exemple, P = phosphoramidite (à titre d'exemple), Hyb1 = première région nucléotidique hybridante comprenant, par exemple, 1-30 bases nucléotidiques, Hyb2 = seconde région nucléotidique hybridante comprenant, par exemple, 1-30 bases nucléotidiques et Hyb3 = troisième région nucléotidique hybridante comprenant, par exemple, 1-30 bases nucléotidiques.

11. - Kit de sondes comprenant des première et seconde sondes d'acide nucléique telles que définies à l'une quelconque des revendications 1 à 10, la première sonde différant de la seconde d'au moins une base d'acide nucléique dans au moins l'une des régions non-contiguës de telle sorte que la première sonde est capable de s'hybrider avec une première séquence cible et la seconde sonde est capable de s'hybrider avec une seconde séquence cible, polymorphe.

12. - Procédé de détection d'une séquence d'acide nucléique cible dans un échantillon comprenant la mise en contact dudit échantillon avec au moins une sonde d'acide nucléique telle que définie à l'une quelconque des revendications 1 à 10 et la détermination de la présence de tout matériel hybridé.

13. - Procédé selon la revendication 12, comprenant une pré-étape d'amplification de l'échantillon d'acide nucléique à l'aide de la réaction en chaîne par polymérase (PCR).

14. - Procédé selon la revendication 12 ou la revendication 13, comprenant une étape de mise en contact de la sonde et de l'acide nucléique échantillon dans des conditions hybridantes.

15. - Procédé selon l'une quelconque des revendications 12 à 14, comprenant une étape de mise en contact des sondes et de l'acide nucléique échantillon dans un protocole d'oligonucléotides spécifiques de la séquence (SSO) standard.

16. - Appareil pour détecter une séquence d'acide nucléique cible dans un échantillon, dans lequel l'appareil comprend une zone d'application de l'échantillon et au moins une sonde telle que définie à l'une quelconque des revendications 1 à 10.

17. - Appareil selon la revendication 16, dans lequel l'appareil est utilisé dans un dosage biologique.

18. - Appareil selon la revendication 17, dans lequel le dosage biologique est utilisé pour la détection d'une ou plusieurs séquences dans un échantillon.

19. - Appareil selon l'une quelconque des revendications 16 à 18, dans lequel le dosage biologique est utilisé pour détecter des polymorphismes dans des antigènes leucocytaires humains (HLA).

20. - Procédé de détection d'une séquence d'acide nucléique cible dans un échantillon, comprenant la mise en contact de l'échantillon avec au moins une sonde telle que définie à l'une quelconque des revendications 1 à 10, et l'opération consistant à déterminer si ou non au moins un hybride sonde/séquence d'acide nucléique cible quelconque est formé.

21. - Procédé de diagnostic comprenant la mise en contact d'une sonde telle que définie à l'une quelconque des revendications 1 à 10, mise au point pour s'hybrider à un allèle ou un nombre d'allèles et/ou de mutations avec un échantillon provenant d'un individu de façon à déterminer le génotype de l'individu.

22. - Procédé de diagnostic selon la revendication 21, dans lequel le procédé de diagnostic est utilisé pour établir l'état des allèles d'antigènes leucocytaires humains dans un individu.
